# EUROPEAN PATENT SPECIFICATION

(11) **EP 3 167 820 B1**
(45) Date of publication and mention of the grant of the patent: **11.12.2024**
(21) Application number: 16201936.8
(22) Date of filing: 08.04.2013
(51) Int. Cl.: A61B 17/115, A61B 17/064

(54) **SURGICAL FASTENER APPLYING APPARATUS**
VORRICHTUNG ZUM ANBRINGEN EINES CHIRURGISCHEN BEFESTIGUNGSELEMENTS
APPAREIL D'APPLICATION D'ATTACHE CHIRURGICALE

(30) Priority: 09.04.2012 US 201213442273
(43) Date of publication of application: 17.05.2017
(62) Divisional of application: 13162779.6
(73) Proprietor: Covidien LP, Mansfield, MA 02048 (US)
(72) Inventor: WILLIAMS, Justin, Southbury, CT 06488 US (US); PENNA, Christopher, Guilford, Connecticut 06437 (US); SCIRICA, Paul, Huntington, Connecticut 06484 (US); RACENET, David, Middletown, Connecticut 06457 (US)
(74) Representative: Morgan, Marc

(56) References cited:
- EP-A2- 2 110 085
- EP-A2- 2 436 318
- US-A- 5 816 471
- US-A1- 2007 131 732
- US-A1- 2007 194 079
- US-A1- 2009 001 121

## Description

### CROSS-REFERENCE TO RELATED APPLICATIONS

The present application is a continuation-in-part of United States Patent Application Serial Number 13/207,653 filed on August 11, 2011, which claims benefit of U.S. Provisional Application No. 61/388,788 filed on October 1, 2010, which claims benefit of U.S. Provisional Application No. 61/410,980 filed on November 8, 2010.

### BACKGROUND

### Technical Field

The present disclosure relates to surgical stapling instruments for applying surgical fasteners or staples to body tissue and, more particularly to surgical stapling instruments utilizing bent backspan staples and having a corresponding anvil including bent staple forming buckets.

### Description of Related Art

Surgical stapling devices for applying an annular array of staples or fasteners to tissue are well known in the art. For example, such surgical stapling devices have particular utility in performing small bowl resection with end-to-end anastomosis. These devices, typically, include a staple pusher assembly or member and an anvil assembly or member at the distal end of the surgical stapling device. The anvil member is movable from a retracted configuration for positioning tissue between the anvil member and the cartridge assembly, to an advanced configuration for joining tissue, i.e., stapling the ends of a tubular organ in a body of the patient organ to be joined. One or more annular or circular arrays of fasteners, such as, for example, staples, is operably housed in the cartridge assembly. The anvil member includes one or more corresponding annular arrays of staple forming bucket members that clinch or form (e.g., in a "B" staple formation) the staples after the staples are expelled from the cartridge assembly. Generally, the staples include a straight backspan. As can be appreciated, the anvil bucket members and/or pushers associated with the cartridge assembly include a corresponding configuration, i.e., a generally straight configuration, to accommodate the straight backspan of the staples.

For a given staple pusher configuration configured for use with straight backspan staples, the number of staples that may be present in a given annular array of staples is limited by the length of the backspan of the staples and an inside and outside diameter of the cartridge assembly. Moreover, it is, typically, an inside annular array of the staples that determine the number of staples that may be present in each additional annular array of staples, e.g., middle and outer annular arrays, of the cartridge assembly. That is, an equal number of staples in each of the annular arrays is, typically, provided to allow for consistent overlap at a gap between each consecutive staple; a specific gap distance exists between each consecutive staple for each annular array of staples, with, typically, the smallest gap distance between each consecutive staple existing in the inner annular array and the gap distance between consecutive staples increasing from the inner annular array to the outer annular array(s). As a result thereof, the gap distances between consecutive staples in the inner and subsequent annular array(s) are unequal. These unequal gap distances are not conducive to obtaining a "tight" staple line. That is, an equal gap distance between consecutive staples in each annular array of staples may promote better healing of the stapled tissue, which, in turn, results in less bleeding and leakage at the stapled tissue line. Unfortunately, the length of the backspan of the aforementioned staples is limited by geometry from interfering with, i.e., extending into, the next row of staples. As a result thereof, subsequent to tissue being stapled with conventional surgical stapling devices, there exists a chance of bleeding and leakage occurring at the stapled tissue line, i.e., adjacent the area between consecutive staples in the annular array of staples in the outer annular array(s). Or, in certain instance, a compromised stapled tissue line being formed, which, in turn, may result in the stapled tissue separating.

Document EP2436318 A2 discloses an anvil assembly, an end effector and/or a surgical stapler suitable for performing curved or circular anastomosis and/or treatment to internal walls of hollow tissue organs wherein the anvil assembly includes an anvil center rod having a proximal end and a distal end, the center rod defining a central longitudinal axis; and an anvil head secured to the distal end of the anvil center rod. The anvil head includes an anvil plate defining a tissue contact surface; and a plurality of staple forming pockets formed in the tissue contact surface of the anvil plate, wherein each of the plurality of staple pockets defines an arcuate longitudinal axis.

Document US5816471 A discloses a surgical stapler with mechanisms for reducing the firing force. This documents discloses a staple that includes a backspan with a lateral displacement, and legs with tips. This feature allows the tips of the staple to avoid contact with the backspan when used with a prior art dimple.

### SUMMARY

According to the invention and as defined in claim 1, a surgical stapler comprises: a tubular body portion; an anvil member disposed at the end of the tubular body portion with straight buckets arranged as an inner and outer annular array; and a surgical staple cartridge assembly disposed at a distal end of the body portion opposite the anvil member. The cartridge has a first inner and a second outer annular array of staples arranged to be ejected therefrom and the straight buckets of the first inner array have a length which is shorter than the length of the buckets in the outer array. The staples have backspans which are generally bent, angled or curved in a plane different to the plane of the legs such that the legs may be crimped to or beyond the backspan when crimped by the straight buckets of the anvil member and such that the staples of the first inner array have a length of backspan which is smaller than the length of the backspan of the outer array such that gaps between each consecutive staple of the first inner array is equal to the gap between each consecutive staple in the outer array and a gap between each consecutive staple forming bucket in the inner annular array of staple forming buckets is equal to a gap between each consecutive staple forming bucket in the outer annular array of the staple forming buckets.

Specific embodiments of the invention are set forth in the dependent claims.

In an aspect of the present disclosure, a surgical stapler comprises: a tubular body portion; a cartridge assembly disposed at a distal end of the body portion for expelling an annular array of staples, each of the staples of the annular array of staples having legsand a generally bent or curved backspan; and an anvil member disposed at the distal end of the tubular body portion and positioned opposite the cartridge assembly to crimp the staples in tissue upon expulsion of the staples from the cartridge assembly, the anvil member having a corresponding annular array of staple forming buckets, each of the buckets having a straight configuration, the staples being crimped by the buckets so that the legs extend to or beyond the backspan.

In certain examples, the cartridge assembly includes an inner annular array of staples and an outer annular array of staples, and the anvil member includes an inner annular array and an outer annular array of staple forming buckets. A length of each of the staples in the inner annular array of staples can be shorter than a length of the each of the staples in the outer annular array of staples and a length of each of the staple forming buckets in the inner annular array of staple forming buckets can be shorter than a length of the each of the staple forming buckets in the outer annular array of staple forming buckets. A gap between each consecutive staple in the inner annular array of staples can be equal to a gap between each consecutive staple in the outer annular array of staples and a gap between each consecutive staple forming bucket in the inner annular array of staple forming buckets can be equal to a gap between each consecutive staple forming bucket in the outer annular array of staple forming buckets.

A gap between each consecutive staple in the inner annular array of staples can be equal to a gap between each consecutive staple in the outer annular array of staples and a gap between each consecutive staple forming bucket in the inner annular array of staple forming buckets can be equal to a gap between each consecutive staple forming bucket in the outer annular array of staple forming buckets.

In certain examples, a length of each of the staples in the inner and outer annular array of staples are equal to one another and a length of each of the staple forming buckets in the inner and outer annular array of staple forming buckets are equal to one another.

In another aspect of the present disclosure, a surgical stapler comprises: a tubular body portion; a cartridge assembly disposed at a distal end of the body portion for expelling inner and outer annular arrays of staples having legs and a generally bent or curved backspan, wherein a length of the backspan of the staples in the outer annular array of staples is greater than a length of the backspan of the staples in the inner annular array of staples; and an anvil member disposed at the distal end of the tubular body portion and positioned opposite the cartridge assembly to crimp the staples in tissue upon expulsion of the staples from the cartridge assembly, the anvil member having corresponding inner and outer annular arrays of staple forming buckets, each of the staple forming buckets having a straight configuration, the staples being crimped by the buckets so that the legs extend to or beyond the backspan.

In certain examples, a gap between each consecutive staple in the inner annular array of staples is equal to a gap between each consecutive staple in the outer annular array of staples and a gap between each consecutive staple forming bucket in the inner annular array of staple forming buckets is equal to a gap between each consecutive staple forming bucket in the outer annular array of staple forming buckets.

In another aspect of the present disclosure, a surgical stapler comprises: a tubular body portion; a cartridge assembly disposed at a distal end of the body portion for expelling a first annular array of staples, each of the staples of the first annular array of staples having a generally straight backspan; and an anvil member disposed at the distal end of the tubular body portion and positioned opposite the cartridge assembly to crimp the staples in tissue upon expulsion of the staples from the cartridge assembly, the anvil member having a corresponding first annular array of staple forming buckets, each of the buckets having a curved or bent configuration such that the annular arrays of staples are crimped beyond the generally straight backspan during formation thereof to provide a first compressive space.

The surgical stapler may have a second annular array of staples and a corresponding second annular array of staple forming buckets, wherein the second annular array of staple forming buckets includes a depth that is less than a depth of the first annular array of staple forming buckets. The formed staples of the second annular array of staples may be crimped to provide a second compressive space that is different than the first compressive space.

In another aspect, a surgical stapler comprises: a tubular body portion; a cartridge assembly disposed at a distal end of the body portion for expelling a first annular array of staples, each of the staples of the first annular array of staples having a generally angled backspan; and an anvil member disposed at the distal end of the tubular body portion and positioned opposite the cartridge assembly to clinch the staples in tissue upon expulsion of the staples from the cartridge assembly, the anvil member having a corresponding first annular array of staple forming buckets, each of the buckets configured to accommodate the generally straight configuration of the staples to facilitate formation thereof such that the annular arrays of staples are crimped beyond the generally angled backspan during formation thereof to provide a first compressive space.

### BRIEF DESCRIPTION OF THE DRAWING

Various embodiments of the present disclosure are described hereinbelow with references to the drawings, wherein:
FIG. 1 is a perspective view of a surgical stapling apparatus including an anvil member and a cartridge assembly according to an embodiment of the present disclosure;
FIG. 2 is a perspective view of the anvil member and cartridge assembly depicted in FIG. 1;
FIG. 3 is a perspective view of a prior art cartridge assembly and anvil member;
FIG. 4 is a perspective view of an enlarged area of detail depicted in FIG. 3;
FIG. 5 is a perspective view of a prior art staple having a straight backspan;
FIG. 6 is a plan view of the cartridge assembly and array of staples contained therein depicted in FIG. 1;
FIG. 7 is a perspective view of the array of staples depicted in FIG. 6;
FIG. 8 is a plan view of the anvil member depicted in FIG. 2;
FIG. 9 is a plan view of an enlarged area of detail depicted in FIG. 8;
FIG. 10 is a plan view of an anvil member according to an alternate embodiment of the present disclosure;
FIG. 11 is a plan view of an enlarged area of detail depicted in FIG. 10;
FIG. 12 is perspective view of a prior art staple;
FIG. 13A is a plan view of an anvil member according to an alternate embodiment of the present disclosure;
FIG. 13B is a plan view of an enlarged area of detail depicted in FIG. 13A;
FIG. 14A is side view of the prior art staple of FIG. 12 shown in a formed configuration;
FIG. 14B is perspective view of the formed staple of FIG. 14A;
FIG. 15A is side view of the prior art staple of FIG. 12 shown in a formed configuration;
FIG. 15B is perspective view of the formed staple of FIG. 15A;
FIG. 16A is perspective view of a staple to be used in an embodiment of the invention;
FIG. 16B is side view of the staple of FIG. 16A shown in a formed configuration; and
FIG. 16C is perspective view of the formed staple of FIG. 16B.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In the drawings and in the descriptions that follow, the term "proximal," as is traditional, will refer to the end of a surgical instrument that is closer to the user, while the term "distal" will refer to the end of the surgical instrument that is farther from the user.

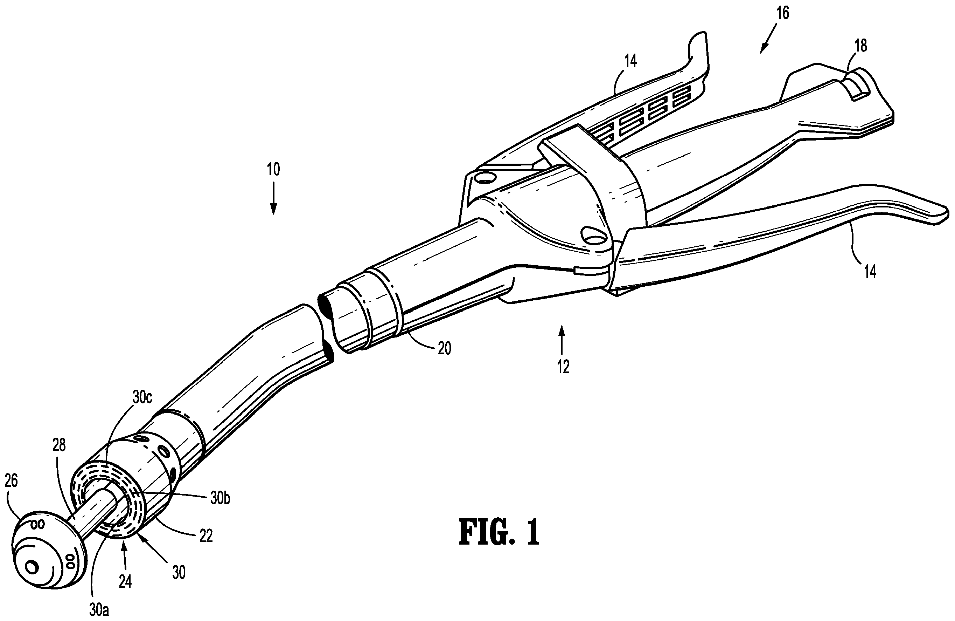

Referring now in specific detail to the drawings, in which like reference numerals identify similar or identical elements throughout the several views, FIG. 1 shows a surgical stapling apparatus 10 (apparatus 10) in accordance with an embodiment of the present disclosure. Apparatus 10 is configured to perform a circular anastomosis of a tubular organ. Briefly, apparatus 10 includes a handle assembly 12 having one or more pivotable actuating handle members 14 (two pivotable handle members 14 shown in the drawings). Apparatus 10 includes an advancing device 16 including a rotatable grip member 18 that is configured to approximate an anvil member 26 towards a cartridge assembly 22. Extending from handle assembly 12 is a tubular body portion 20 that includes a generally curved configuration. In certain embodiments, body portion 20 may also be straight and, in other embodiments, may be flexible to bend to any configuration. Body portion 20 terminates in cartridge assembly 22 that is associated with an annular array of staples 24, see FIGS. 1, 6 and 7. Anvil member 26 is positioned opposite cartridge assembly 22 and is connected to apparatus 10 by shaft 28 at connection device or structure (not explicitly shown) operably disposed within the cartridge assembly 22. For a more detailed description of the body portion 20, advancing device 16, handle assembly 12 including handle members 14 reference is made to commonly-owned U.S. States Patent No. 5,915,616 to Viola et al., filed on October 10, 1997. For example, the tubular body portion 20 can include a shaft for connection to the shaft 28 and the rotatable grip member 18 when rotated moves the shaft of the body portion 20 and approximates the anvil member 26 with the staple cartridge assembly 22. The stapling apparatus 10 further includes a pusher member, having a plurality of fingers for advancing the staples out of the cartridge assembly 22 and toward the anvil member. Movement of the pivoting actuating handle members 14 moves the pusher member to eject the staples. The handle assembly 12 includes assemblies for moving the shaft of the body portion 20 and pusher member. For example, a cam member having a helical groove receives a pin of the grip member 18 so that as the grip member is rotated, the cam member moves proximally, moving the shaft of the body portion 20. Threaded members and other means can be used to actuate the pusher member and move the anvil member 26 toward and away from the cartridge assembly 22. It is contemplated that the cartridge assembly is a removable and replaceable unit, so that the stapling apparatus 10 can be reloaded and used again.

It is also contemplated that the apparatus has a replaceable head including the cartridge assembly, anvil member and associated mechanisms. The stapling apparatus 10 can include the manually actuated handle assembly of FIG. 1 and as described above, or can include a powered actuator assembly having first and second drive members. For example, U.S. Patent Application No. 12/946,082, filed November 15, 2010, discloses a surgical device having a powered actuator assembly. Such actuator assembly can be powered by a motorized handle.

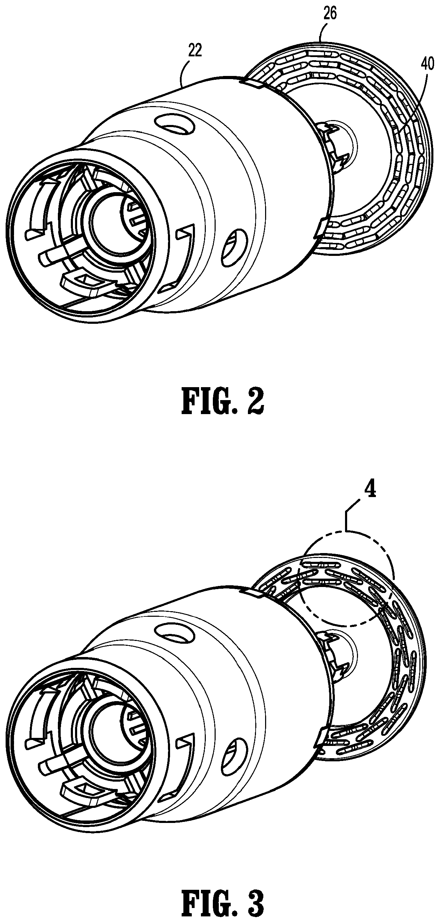

With reference to now to FIGS. 2, 6-9, anvil member 26 and cartridge assembly 22 according to an embodiment of the present disclosure is illustrated.

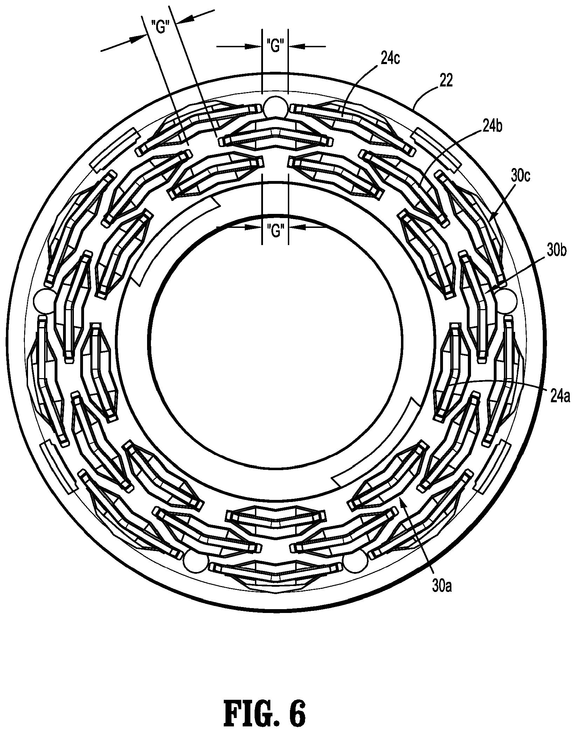

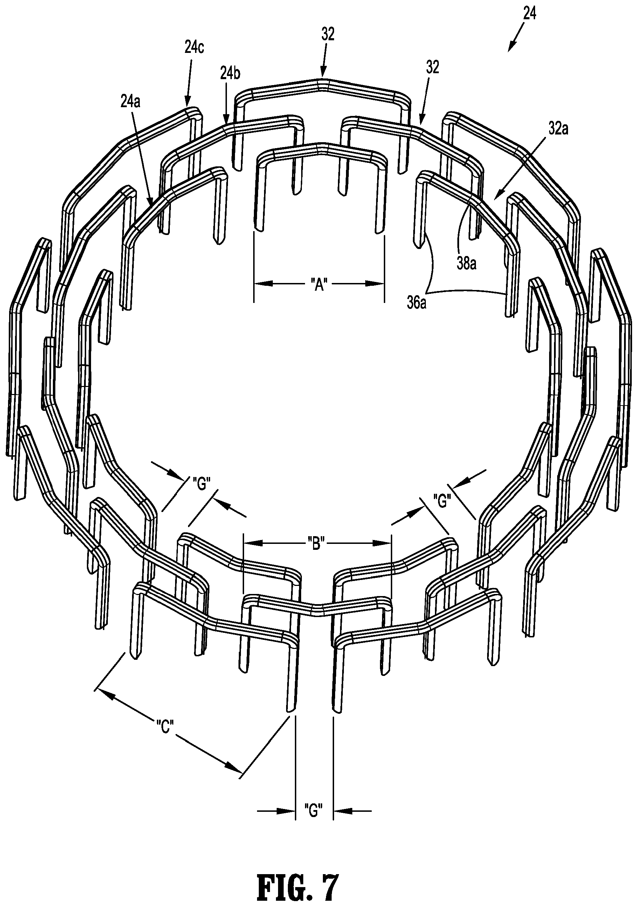

Cartridge assembly 22 is configured to house a plurality of staples 24 (FIGS. 1, 6 and 7). In particular, cartridge assembly 22 includes an array of annular slots 30 (FIGS. 1 and 6) that are configured to house a corresponding annular array of staples 24 (FIGS. 6 and 7). In the illustrated embodiment, there are three annular arrays of slots 30 including an inner annular array of slots 30a, a middle annular array of slots 30b and an outer annular array of slots 30c (collectively referred to herein as slots 30 unless otherwise noted) and three corresponding annular arrays of staples including an inner annular array of staples 24a, a middle annular array of staples 24b and an outer annular array of staples 24c (collectively referred to herein as staples 24 unless otherwise noted), see FIG. 6. In certain embodiments, cartridge assembly 22 and anvil member 26 may include two annular arrays of staples and corresponding slots.

Slots 30 are aligned with a plurality of corresponding staple pushers (not explicitly shown). In certain embodiments, the staple pushers include a generally bent configuration to facilitate expelling the staples 30 from the cartridge assembly 22. In other embodiments, the staple pushers may be configured with other configurations, i.e., straight, rounded, etc.

Staples 24 may be made from any suitable biocompatible material including, but not limited, to surgical steel, shape memory alloys, polymeric materials, etc. In the illustrated embodiment, the staples 24 are made from surgical steel. In certain embodiments, it may prove advantageous to have one or more annular array of staples, e.g., inner annular array of staples 24a, made from one material and one or more annular array of staples, e.g., middle annular array of staples 24b and outer annular array of staples 24c, made from a different material.

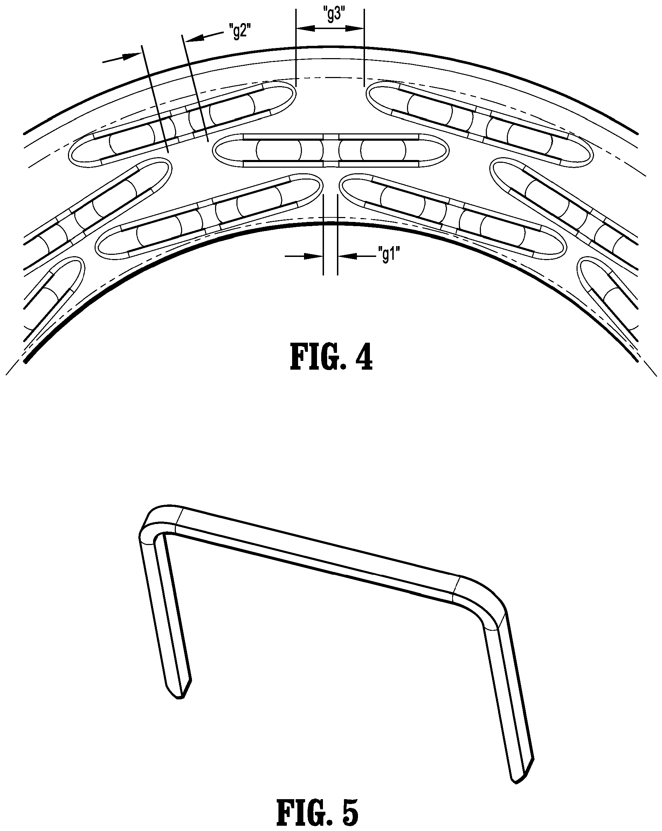

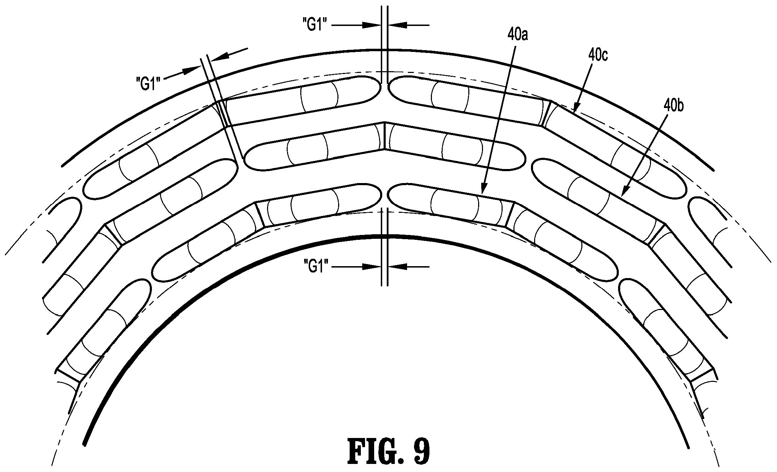

Staples 24 are similar to conventional staples, however, unlike conventional staples (FIG. 5), staples 24 include a backspan 32 having a generally bent or angled configuration, as best seen in FIG. 7. The bent backspan of the staples 24 allows the staples to be arranged such that an equal or consistent gap distance "G" between each consecutive staple in the inner annular array of staples 24a, middle annular array of staples 24b, and outer annular array of staples 24c is achievable (FIG. 9), as described in greater detail below.

Continuing with reference to FIG. 7, the operative features of the staples 24 are described in terms of staples 24a of the inner annular array of staples 24a. Staples 24a include a pair of legs 36a having a generally pointed tip (as best seen in FIG. 7), although the tip may include other suitable configurations, e.g., blunt, flat, beveled, etc. Legs 36a extend from a backspan 32a.

Backspan 32a includes a generally bent or curved configuration, wherein a radius of curvature of the backspan 32a is greatest at a medial portion 38a (FIG. 7). The radius of curvature at the medial portion 38a may be adjusted to accommodate specific surgical procedures, specific surgical devices, a number of annular arrays of staples, a manufacturer's contemplated uses, etc. The bent backspan 32a of the staples 24a allows the staples 24a to be arranged in the inner annular array staples 24a with a minimum gap distance "G" between each consecutive staple 24a. That is, unlike conventional staples with straight backspans, the bent backspan 32a of the staple 24a is configured to follow a contour of the cartridge assembly 22 and, thus, allow a "tighter" grouping of the staples 24a (i.e., a higher density of staples) in the inner annular array of staples 24a. Moreover, a bent backspan 32b and 32c of the staples 24b and 24c, respectively, allows the lengths of the staples in these annular arrays to be larger than the lengths of the staples 24a in the annular array of staples 24a. That is, because of the bent backspans 32b and 32c of respective staples 24b and 24c, a length of the staples 24b and 24c can be increased to accommodate "overlapping" of specific gap distances "G" between consecutive staples 24a in the annular array of staples 24a. For example, and with specific reference to FIG. 7, each staple 24a in the inner annular array of staples 24a includes a length "A" and a gap between each consecutive staple 24a in the inner annular array of staples 24a is equal to gap "G." The length of staples 24b in the middle annular array of staples 24b includes a length "B" that is larger than the length "A" of the staples 24a, and a gap between each consecutive staple 24b in the middle annular array of staples 24b is equal to the gap "G" (see FIG. 7). The length of staples 24c in the outer annular array of staples 24c includes a length "C" that is larger than the lengths "A" and "B" of the respective staples 24a and 24c, and a gap between each consecutive staple 24c in the outer annular array of staples 24c is equal to the gap "G" (see FIG. 7).

As can appreciated, the "tighter" grouping of the staples 24a-24c allows the staples to "nested" and, thus, more closely packed together with respect to one another for a given cartridge assembly 22 when compared to staples with straight backspans. This "nested" configuration of the staples 24a-24c provides an increased inside diameter of staples 24a when compared to staples with straight backspans.

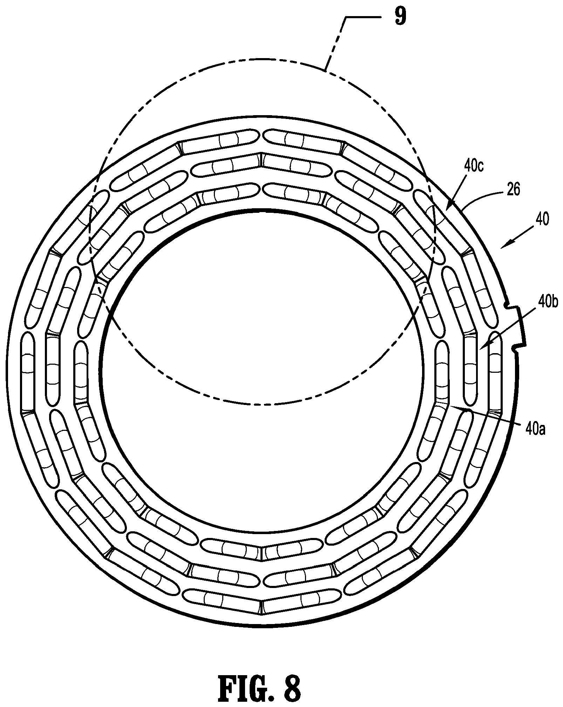

With reference now to FIGS. 8 and 9, anvil member 26 includes corresponding annular arrays of staple forming buckets 40 including an inner annular array of staple forming buckets 40a, a middle annular array of buckets 40b and an outer annular array of buckets 40c. Unlike conventional staple forming buckets (FIGS. 3 and 4), each of the staple forming buckets 40a, 40b and 40c are configured to accommodate the generally bent configuration of the corresponding staples 24a, 24b and 24c to facilitate formation thereof. To this end, each of the buckets 40a, 40b and 40c include a generally bent or curved configuration and is proportioned to respective staples 24a, 24b and 24c therein such that the staples 24a, 24b and 24c have a generally "B" configuration upon formation thereof. A radius of curvature of the staple forming buckets 40a, 40b and 40c is greatest at a medial portion 38a to match the radius of curvature of the corresponding staples 24a, 24b and 24c.

Continuing with reference to FIGS. 8 and 9, the dimensions of the staple forming bucket 40a in the inner annular array of staple forming buckets 40a is substantially equal to the dimensions of the corresponding staples 24a. In particular, the staple forming bucket 40a is slightly larger than the staple 24a to facilitate forming the staple 24a into the "B" formation. A gap between each consecutive staple forming bucket 40a in the inner annular array of staple forming bucket 40a is equal to gap "G₁." In the illustrated embodiment, the gap "G₁" is illustrated less than the gap "G" as a result of the staple forming bucket 40a being larger than the staple 24a. Alternatively, and in certain embodiments, the gap "G₁" may be equal to the gap "G." Similarly, the dimensions of the staple forming buckets 40b and 40c of the middle and outer annular arrays of staple forming buckets 40b and 40c are substantially equal to the dimensions of the corresponding staples 24b and 24c of the middle and outer annular arrays of staples 24b and 24c. The gap between each consecutive staple forming buckets 40b and 40c is equal to the gap "G₁" between each consecutive staple forming bucket 40a, see FIG. 9.

In use, tissue, e.g., a portion of a tubular organ, is positioned between the anvil member 26 and cartridge assembly 22. Rotatable grip 18 of the advancing device 16 is actuated to approximate the anvil member 26 towards the cartridge assembly 22. Handles 14 may be pivoted to drive or expel the staples 24 through the tissue against the anvil member 26 to complete a circular anastomosis of a tubular organ.

In accordance with the present disclosure, the annular arrays of formed staples 24a, 24b and 24c form a "tight" staple line by virtue of the consistent gap "G" between consecutive staples 24a, 24b and 24c in the inner, middle and outer annular arrays of staples 24a, 24b and 24c, and the likelihood of bleeding or leaking occurring between consecutive staples 24a, 24b and 24c in the inner, middle and outer annular arrays of staples 24a, 24b and 24c is reduced, if not eliminated. The unique bent configuration of the staples 24a, 24b and 24c and corresponding staple forming buckets 40a, 40b and 40c overcomes the aforementioned drawbacks typically associated with conventional surgical stapling devices. That is, gaps "g1," "g2," and "g3" (FIG. 3 and 4) between consecutive staple forming buckets in respective inner, middle and outer annular arrays of conventional surgical stapling devices, increase from the inner annular array toward the outer annular array, i.e., "g1"<"g2"<"g3;" as can be appreciated, the formed staples in tissue will be spaced apart from one another at a distance that corresponds to the gap distances "g1," "g2," and "g3, of the staple forming buckets. As noted above, these "unequal" gap distances are not conducive in obtaining a "tight" staple line.

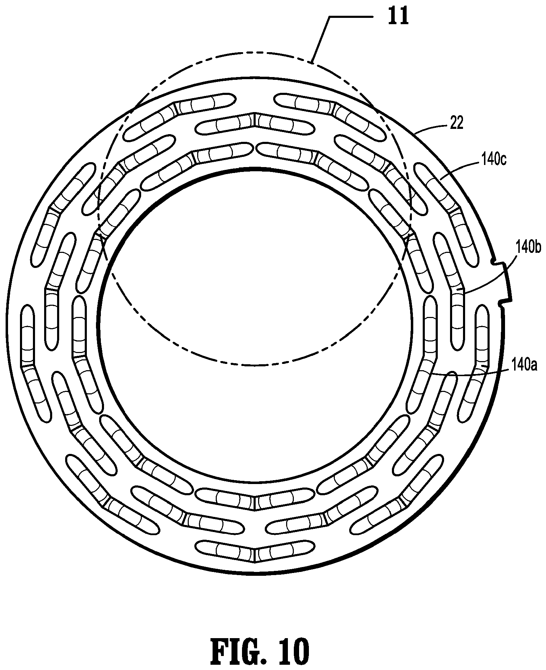

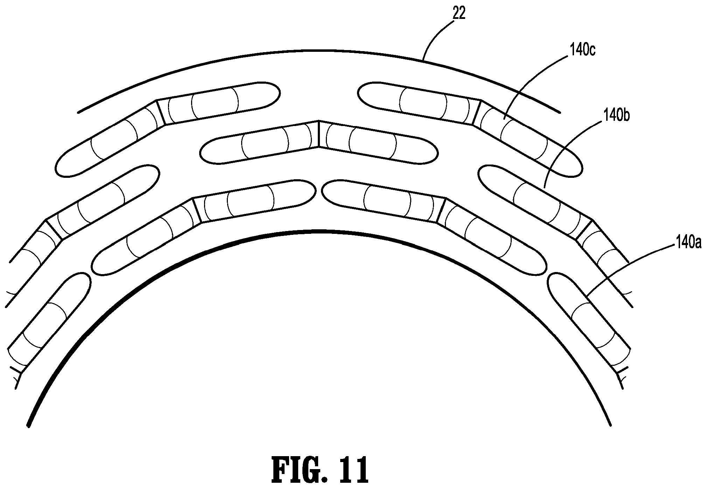

From the foregoing and with reference to the various figure drawings, those skilled in the art will appreciate that certain modifications can also be made to the present disclosure without departing from the scope of the same. For example, while the staples 24a, 24b and 24c and corresponding staple forming buckets 40a, 40b and 40c have been described herein as having different lengths, it is within the purview of the present disclosure that the staples 24a, 24b and 24c and corresponding staple forming buckets 40a, 40b and 40c may have the same lengths (FIGS. 10 and 11). In this embodiment, the staples (not explicitly shown) and corresponding staple forming buckets 140 including inner, middle and outer annular arrays of staple forming buckets 140a-140c have the same length. In the embodiment illustrated in FIGS. 10 and 11, an equal number of staple forming buckets 140 are shown in the inner annular array of staple forming buckets 140a, middle annular array of staple forming buckets 140b and outer annular array of staple forming buckets 140c. This embodiment may prove advantageous where a "tight" staple line is not required.

In the instance where a "tight" staple line is required, however, the unique bent backspan of the staples and corresponding staple forming buckets 140b-140c allows for one or more extra staples (or larger staples) and corresponding staple forming buckets 140b-140c to be provided in the middle and outer annular arrays of staples and corresponding staple forming buckets 140b-140c. That is, for a given cartridge assembly and anvil, the geometry, e.g., bent backspan, of the staples and corresponding staple forming buckets 140b-140c follows a contour of the cartridge assembly and anvil, respectively, and, as a result thereof, allows extra staples and staple forming buckets 140b to be provided in the annular array of staple forming buckets 140b without interfering with staples and staple forming buckets 140c. And, likewise, allows extra staples and staple forming buckets 140c to be provided in the annular array of staple forming buckets 140c without interfering or extending into an outer peripheral edge of the cartridge assembly and anvil. The extra staples and corresponding staple forming buckets 140a-140c may be provided to sufficiently overlap the gaps between consecutive staples and staple forming buckets 140a-140c.

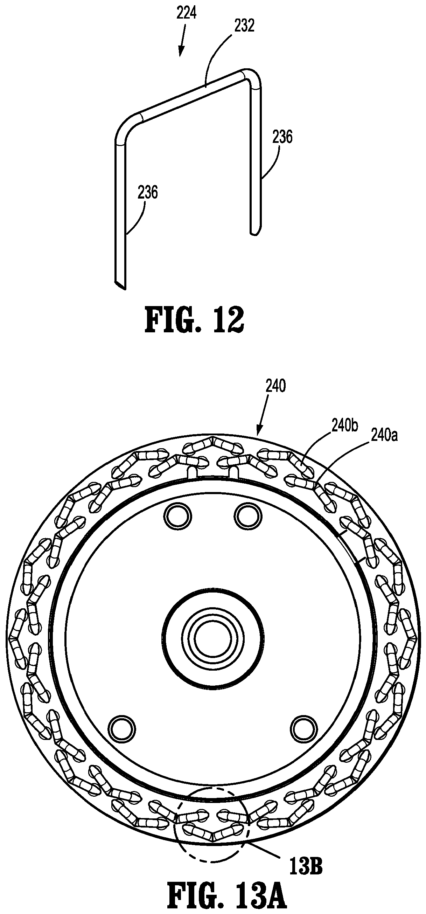

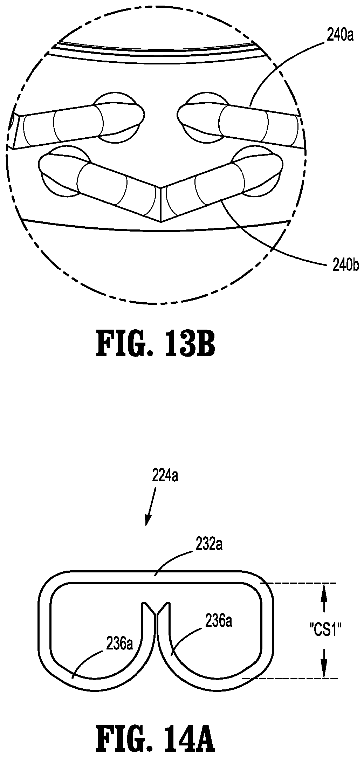

With reference to FIGS. 12-15B an alternate embodiment of the staple and staple forming buckets are illustrated designated 224 (FIG. 12) and 240 (FIGS. 13A and 13B), respectively. Only those features unique to staples 224 and staple forming buckets 240 are discussed herein.

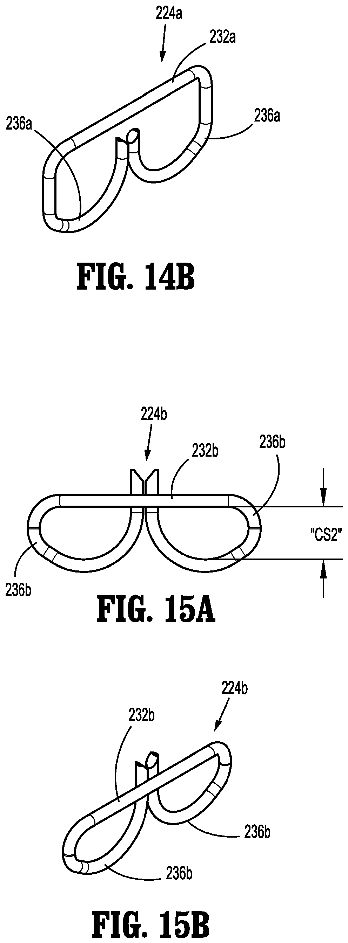

In the embodiment illustrated in FIGS. 12-15B, staples 224 and staple forming buckets 240 may be utilized to provide formed staples with different internal spaces for compressing tissue to achieve a desired level of hemostasis and blood flow in stapled tissue segments. In one particular embodiment, for example, an inner annular row of staples 224a (staples 224a) may provide a greater compressive space (FIGS. 14A and 14B) for stapling tissue than an outer annular row of staples 224b (staples 224b), see FIGS. 15A and 15B. In other words, staples 224b in a formed configuration provide a greater compressive force to stapled tissue than the staples 224a in a formed configuration. Thus, because a pressure exerted on tissue stapled by staples 224b is greater than the pressure exerted on tissue stapled by staples 224a, the blood flow through the tissue surrounding staples 224b will be less (more restricted) than the blood flow through the tissue surrounding staples 224a, thereby further facilitating hemostasis. However, because blood flow is not completely restricted through tissue stapled by staples 224b, blood perfusion is improved and unnecessary necrosis of the stapled tissue may be prevented and/or impeded.

Staples 224a and 224b include respective staple legs 236a (FIGS. 14A-14B) and 236b (FIGS. 15A-15B) that extend from backspans 232a, 232b. In the embodiment illustrated in FIGS. 12-15B, staple legs 236a and 236b have the same length as each other, and backspans 232a, 232b include a "straight" configuration to facilitate forming staples 224a, 224b in the staple forming buckets 240a, 240b (FIGS. 13A and 13B).

Staple forming buckets 240 are described herein in terms of inner annular row of staple forming buckets 240a (buckets 240a) and outer annular row of staple forming buckets 240b (buckets 240a), see FIGS. 13A and 13B. As can be appreciated, greater or fewer rows of buckets 240 may be utilized.

Each of buckets 240a and 240b are configured to receive corresponding staples 224a and 224b therein to form the staples 224a and 224b. Buckets 240a and 240b include an angled configuration to facilitate forming the respective staples 224a and 224b. Unlike the previously described staple forming buckets, however, buckets 240a include depth that is different from a depth of buckets 240b to facilitate forming the staples 224a and 224b with different compressive spaces. For example, and in one particular embodiment, buckets 240a include a depth that is greater than a depth of the buckets 240b to form the staples 224a with a compressive space "CS1" (FIG. 14A) that is greater than a compressive space "CS2" (FIG. 15A) of the staples 224b. Specifically, the angled buckets 240a, 240b having different depths in combination with the staples 224a, 224b having respective straight backspans 232a, 232b and legs 236a and 236b with the same length allows the staples 224a, 224b to formed with different compressive spaces, see FIGS. 14B and 15B for example. That is, this specific combination of staples 224a, 224b and buckets 240a, 240b allows staples 224b to be crimped beyond the backspan 232b (FIGS. 15A-15B) to provide a compressive space "CS2" that is less than a compressive space "CS1" provided by the staples 224a such that tissue stapled by staples 224b is under greater pressure than tissue stapled by staples 224a. In this way, the backspan does not interfere with the deformation of the legs of the staples, and the degree of crimping can be varied. The staples can be crimped by the buckets so that the legs of the staples extend to or beyond the backspan without interference from the backspan.

In certain embodiments, it may prove advantageous to have the staples 224a exert a greater pressure to stapled tissue than the staples 224b. In this embodiment, buckets 240a will include a depth that is less than a depth of the buckets 240b.

In certain embodiments, each of the staples 224a, 224b may be crimped beyond the respective backspans 232a, 232b. For example, the buckets 240a may have a depth that is configured to crimp the staples 224a beyond the backspan 232a such that the compressive space provided therefrom is greater than or less than the compressive space provided by staples 224b.

In one particular embodiment, the buckets 240a, 240b may have the same depth and the staple legs 236a, 236b may have different lengths to achieve the aforementioned compressive spaces. Those skilled in the art will appreciate the various lengths of the staple legs 236a, 236b that will be needed to achieve a specific compressive space when the staples 224a and 224b are formed.

In certain embodiments, the operation of the advancing device 16, the pusher member, or both, can be utilized to vary the degree to which the staples are deformed or crimped. For example, by approximating the anvil member more closely with the cartridge assembly, the staples are crimped with a relatively smaller internal space and the tissue is compressed to a greater degree. Alternatively, the pusher member can be further advanced, further crimping or deforming the staples. Desirably, there is an indicator on the stapling apparatus handle assembly 12 that allows the surgeon to gauge the degree to which the staples will be crimped. The varyable crimp can be used in any of the embodiments disclosed herein, including embodiments in which different sized (preformation) staples are used, same sized staples are used, bent or curved backspan staples are used, and/or bent or curved staple forming buckets are used.

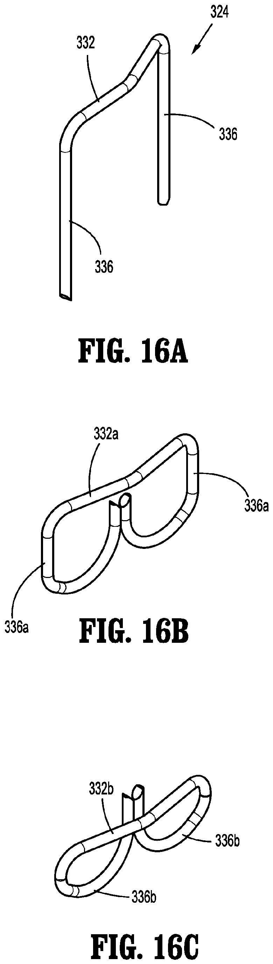

In some embodiments, such as the one illustrated in FIGS. 16A-16C, the buckets (not explicitly shown) may have a generally straight configuration and the staples 324 may include a backspan 332 having a generally angled configuration to facilitate crimping the staples 324 beyond the backspan 332. In this way, the backspan does not interfere with the deformation or crimping of the legs of the staples. The staples can be crimped by the buckets so that the legs extend to or beyond the backspan, without interference from the backspan. In one particular embodiment, for example, a formed staple 324a with legs 336a extending from an angled backspan 332a may provide a compressive space that is similar to compressive space "CS1" (FIG. 16B) and formed staple 324b with legs 336b extending from an angled backspan 332b may provide a compressive space that is similar to compressive space "CS2" (FIG. 16C). The straight configuration of the buckets can be used with any of the embodiments disclosed herein, including stapling apparatus that vary the degree of crimping or deformation of the staples, as well as embodiments in which different sized (preformation) staples are used, or same sized staples are used.

It is also contemplated that the stapling apparatus can be configured to apply three rows of staples, and that the staples can have more than one bend in the backspan, or a curved backspan that is irregular (i.e., having more than one radius), in any of the embodiments disclosed herein.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

## Claims

1. A surgical stapler, comprising:
a tubular body portion;
an anvil member disposed at the end of the tubular body portion with straight buckets arranged as an inner and outer annular array; and
a surgical staple cartridge assembly disposed at a distal end of the body portion opposite the anvil member which cartridge having a first inner and a second outer annular array of staples arranged to be ejected therefrom
wherein the straight buckets of the first inner array have a length which is shorter than the length of the buckets in the outer array and
wherein the staples have backspans which are generally bent, angled or curved in a plane different to the plane of the legs such that the legs may be crimped to or beyond the backspan when crimped by the straight buckets of the anvil member and such that the staples of the first inner array have a length of backspan which is smaller than the length of the backspan of the outer array such that gaps between each consecutive staple of the first inner array is equal to the gap between each consecutive staple in the outer array and a gap between each consecutive staple forming bucket in the inner annular array of staple forming buckets is equal to a gap between each consecutive staple forming bucket in the outer annular array of the staple forming buckets.

2. A surgical stapler as claimed in claim 1 wherein the staples of the first annular array is made of a first material and the staples of the second annular array are made of a second material different to the first material

3. A surgical stapler as claimed in claim 1 or 2 wherein the backspans have a curvature which is greatest at a medial portion.

4. A surgical stapler as claimed in any claim 1, 2 or 3 wherein the staple backspan is configured to allow the staple to, in use, follow a contour of the surgical staple cartridge assembly.

5. A surgical stapler as claimed in any preceding claim wherein the staple is made from a biocompatible material.

6. A surgical stapler as claimed in any preceding claim wherein the biocompatible material is one of surgical steel, shape memory alloy, polymeric materials.

7. A surgical stapler as claimed in any preceding claim wherein the legs are provided with a tip at their distal end which is formed as one of a blunt tip, a generally pointed tip, a flat tip or a bevelled tip.

## Patentansprüche

1. Chirurgische Klammervorrichtung, umfassend:
einen röhrenförmigen Körperabschnitt;
ein Ambosselement, das am Ende des röhrenförmigen Körperabschnitts angeordnet ist und gerade Behälter hat, die als eine innere und äußere ringförmige Anordnung angeordnet sind; und
eine chirurgische Klammermagazinanordnung, die an einem distalen Ende des Körperabschnitts gegenüber dem Ambosselement angeordnet ist, wobei das Magazin eine erste innere und eine zweite äußere ringförmige Anordnung von Klammern hat, die dazu angeordnet sind, daraus ausgestoßen zu werden
wobei die geraden Behälter der ersten inneren Anordnung eine Länge haben, die kürzer ist als die Länge der Behälter in der äußeren Anordnung und
wobei die Klammern Brückenstege haben, die im Allgemeinen gebogen, winklig oder gekrümmt sind in einer Ebene, die sich von der Ebene der Schenkel unterscheidet, so dass die Schenkel an den Brückensteg oder darüber hinaus gecrimpt werden können, wenn sie von den geraden Behältern des Ambosselements gecrimpt werden, und so dass die Klammern der ersten inneren Anordnung eine Länge des Brückenstegs haben, die kleiner ist als die Länge des Brückenstegs der äußeren Anordnung, so dass die Lücken zwischen jeder nachfolgenden Klammer der ersten inneren Anordnung gleich der Lücke zwischen jeder nachfolgenden Klammer in der äußeren Anordnung ist und eine Lücke zwischen jedem nachfolgenden klammerbildenden Behälter in der inneren ringförmigen Anordnung von klammerbildenden Behältern gleich einer Lücke zwischen jedem nachfolgenden klammerbildenden Behälter in der äußeren ringförmigen Anordnung der klammerbildenden Behälter ist.

2. Chirurgische Klammervorrichtung nach Anspruch 1, wobei die Klammern der ersten ringförmigen Anordnung aus einem ersten Material hergestellt sind und die Klammern der zweiten ringförmigen Anordnung aus einem zweiten Material hergestellt sind, das sich von dem ersten Material unterscheidet.

3. Chirurgische Klammervorrichtung nach Anspruch 1 oder 2, wobei die Brückenstege eine Krümmung haben, die an einem mittleren Abschnitt am größten ist.

4. Chirurgische Klammervorrichtung nach Anspruch 1, 2 oder 3, wobei der Klammerbrückensteg dazu ausgelegt ist, zu ermöglichen, dass die Klammer, in Verwendung, einer Kontur der chirurgischen Klammermagazinanordnung folgt.

5. Chirurgische Klammervorrichtung nach einem vorhergehenden Anspruch, wobei die Klammer aus einem biokompatiblen Material hergestellt ist.

6. Chirurgische Klammervorrichtung nach einem vorhergehenden Anspruch, wobei das biokompatible Material eins von chirurgischem Stahl, Formgedächtnislegierung, polymeren Materialien ist.

7. Chirurgische Klammervorrichtung nach einem vorhergehenden Anspruch, wobei die Schenkel an ihrem distalen Ende mit einer Spitze bereitgestellt sind, die als eine von einer stumpfen Spitze, einer im Allgemeinen spitzen Spitze, einer flachen Spitze oder einer abgeschrägten Spitze gebildet ist.

## Revendications

1. Agrafeuse chirurgicale comprenant :
une partie de corps tubulaire ;
un élément d'enclume disposé à l'extrémité de la partie de corps tubulaire avec des godets droits agencés en un réseau annulaire intérieur et extérieur ; et
un ensemble de cartouche d'agrafes chirurgicales disposées à une extrémité distale de la partie du corps opposée à l'enclume, cette cartouche comportant un premier réseau annulaire intérieur et un second réseau annulaire extérieur d'agrafes agencées pour être éjectées de celle-ci
les godets droits de la première rangée intérieure ayant une longueur qui est inférieure à celle des godets de la rangée extérieure, et
les agrafes ayant des dos qui sont généralement courbés, coudés ou incurvés dans un plan différent de celui des pattes, de sorte que les pattes peuvent être serties jusqu'à ou au-delà du dos lorsqu'elles sont serties par les godets droits de l'élément d'enclume et de sorte que les agrafes du premier réseau intérieur ont une longueur de dos qui est inférieure à la longueur du dos du réseau extérieur, de sorte que l'écart entre chaque agrafe consécutive du premier réseau intérieur soit égal à l'écart entre chaque agrafe consécutive du réseau extérieur et qu'un écart entre chaque godet de formation d'agrafes consécutif dans le réseau annulaire intérieur de godets de formation d'agrafes soit égal à un écart entre chaque godet de formation d'agrafes consécutif dans le réseau annulaire extérieur de godets de formation d'agrafes.

2. Agrafeuse chirurgicale selon la revendication 1, les agrafes du premier réseau annulaire étant constituées d'un premier matériau et les agrafes du second réseau annulaire étant constituées d'un second matériau différent du premier matériau.

3. Agrafeuse chirurgicale selon la revendication 1 ou 2, les dos ayant une courbure qui est la plus importante au niveau de la partie médiane.

4. Agrafeuse chirurgicale selon l'une quelconque des revendications 1, 2 ou 3, le dos de l'agrafe étant configuré pour permettre à l'agrafe de suivre, lors de l'utilisation, un contour de l'ensemble de cartouche d'agrafes chirurgicales.

5. Agrafeuse chirurgicale selon l'une quelconque des revendications précédentes, l'agrafe étant constituée d'un matériau biocompatible.

6. Agrafeuse chirurgicale selon l'une quelconque des revendications précédentes, le matériau biocompatible étant un acier chirurgical, un alliage à mémoire de forme ou un matériau polymère.

7. Agrafeuse chirurgicale selon l'une quelconque des revendications précédentes, les pattes étant pourvues d'une pointe à leur extrémité distale, formée d'une pointe émoussée, d'une pointe généralement pointue, d'une pointe plate ou d'une pointe biseautée.
